# EUROPÄISCHE PATENTSCHRIFT

(11) **EP 1 322 988 B1**
(45) Veröffentlichungstag und Bekanntmachung des Hinweises auf die Patenterteilung: **07.04.2004**
(21) Anmeldenummer: 01911450.3
(22) Anmeldetag: 13.02.2001
(51) Int. Cl.: G02B 25/02

(54) **DERMATOLOGIE-HANDLUPE**
DERMATOLOGICAL HAND GLASS
LOUPE A MAIN A USAGE DERMATOLOGIQUE

(30) Priorität: 11.03.2000 DE 10011351; 27.04.2000 DE 10020715
(43) Veröffentlichungstag der Anmeldung: 02.07.2003
(73) Patentinhaber: LINOS Photonics GmbH & Co. KG, 37081 Göttingen (DE)
(72) Erfinder: MATERN, Ulrich, 82362 Weilheim (DE); PARTHEYMÜLLER, Ulrich, 83607 Holzkirchen (DE); BÜRCKNER-KOYDL, Dieter, 86415 Mering (DE); APITZSCH, Jürgen, 81827 München (DE); KNOCH, Stefan, 81547 München (DE); ROTHE, Martin, 81379 München (DE)
(74) Vertreter: Säger, Manfred, Dipl.-Ing.
(86) Internationale Anmeldenummer: PCT/DE2001/000544
(87) Internationale Veröffentlichungsnummer: WO 2001/069301

(56) Entgegenhaltungen:
- CH-A- 262 015
- DE-U- 9 000 190
- DE-U- 9 412 153
- DE-U- 29 923 590
- FR-A- 652 239
- US-A- 5 618 289

## Beschreibung

### Technisches Gebiet

Die Erfindung bezieht sich auf eine Dermatologie-Handlupe gemaß dem Oberbegriff des Hanptanspruchs.

Derartige Systeme werden beispielsweise zur Untersuchung pigmentierter Hautveränderungen zur Früherkennung des malignen Melanoms eingesetzt. Für weitere Anwendungen derartiger Systeme wird auf die einschlägige medizinische Literatur verwiesen.

### Stand der Technik

Dermatologie-Handlupen gemäß dem Oberbegriff des Patentanspruchs 1 sind allgemein bekannt und werden von einer Reihe von Herstellern hergestellt.

Die bekannten Dermatologie-Handlupen weisen eine Reihe von Nachteilen auf:

Die Beleuchtung erfolgt meist über eine Halogenlampe mit oder ohne nachgeordnete Lichtleiter. Aufgrund dieser Ausbildung ist die Beleuchtung des zu untersuchenden Hautbereichs nicht sehr gleichmäßig. Zudem ist die spektrale Verteilung des Lichts einer Halogenlampe alterungsabhängig und variiert darüberhinaus von Hersteller zu Hersteller und von Charge zu Charge. Dies erschwert die Farbbeurteilung von Hautstellen.

Verschiedene der bekannten Handlupen sind zwar modular aufgebaut, der Auseinanderbau und das Einsetzen anderer Teile ist jedoch bei manchen Lupen kompliziert.

Zudem ist die Größenbeurteilung aufgrund undefinierter Einsatzbedingungen schwierig.

Auf die bekannten Systeme - wie sie beispielsweise von den Firmen Heine Optotechnik, Herrsching oder unter dem Namen "EpiScope" von Welch Allyn vertrieben werden - wird im übrigen zur Erläuterung aller hier nicht näher beschriebenen Einzelheiten und Anwendungsmöglichkeiten ausdrücklich verwiesen (DE G 90 00 190).

Es ist ferner ein optischer Betrachtungs- und Beleuchtungsapparat mit einer zentralen Lupe und einer darum angeordneten Beleuchtungseinheit bekannt (CH-262015), bei der das Licht mittels Sammellinsen in Form von Lichtkegeln an der Untersuchungsstelle mehr oder weniger verstärkt oder nach Wunsch auf bestimmte Bereiche gelenkt werden kann. Als Lichtquellen dienen hierbei bogenförmig ausgebildete Beleuchtungsröhren.

### Darstellung der Erfindung

Der Erfindung liegt die Aufgabe zugrunde, eine Dermatologie-Handlupe gemaß dem Obebegriff des hanptanspruchs anzugeben, bei dem der Abbildungsmaßstab des betrachteten genau definiert und exakt reproduzierbar ist und/oder die Farbe des Bildes genau wiedergegeben wird, so daß Vergleiche mit zu anderen Zeiten und/oder mit anderen Systemen betrachteten Bildern möglich sind, und/ oder die Lupe klein und kompakt und ergonomisch günstig handhabbar ist.

Eine erfindungsgemäße Lösung dieser Aufgabe ist in dem unabhängigen Hanptanspruch angegeben. Weiterbildungen der Erfindung sind Gegenstand der abhängigen Ansprüche.

Bei der Lösung der erfindungsgemäßen Aufgabenstellung weist die Beleuchtungseinheit als Lichtquelle wenigstens eine Weißlicht-Leuchtdiode auf. Leuchtdioden unterliegen keiner Alterung; zudem haben die einzelnen Leuchtdioden ein wesentlich gleichmaßigeres Spektrum als beispielsweise Halogenlampen. Geeignete Leuchtdioden werden beispielsweise von der Firma SLOAN unter der Bezeichnung L3W34NO vertrieben. Eine besonders gleichmäßige Beleuchtung - die insbesondere der Beleuchtung mit Lichtleitern deutlich überlegen ist - erhält man ferner bei einer Mehrzahl von Weißlicht-Leuchtdioden, die ringförmig um die Lupenoptik angeordnet sind. Bei dem erfindungsgemäßen System kann die Gleichmäßigkeit der Beleuchtung größer als 90 % sein. Dieser Wert ist deutlich größer als beim Stand der Technik. Die Gleichmäßigkeit der Beleuchtung wird dadurch verbessert daß das Licht der Weißlicht-Leuchtdioden auf eine mattierte Fläche gerichtet wird, die das Licht auf den zu beleuchtenden Hautbereich reflektiert, wobei diese mattierte Fläche die Innenseite des jeweils verwendeten Abstandsaufsatzes ist. Diese Ausbildung hat den Vorteil, daß durch das Auswechseln der Abstandsaufsätze auch der Kegel des Beleuchtungslichts angepaßt wird.

Ferner kann in zweckmäßiger Weiterbildung des Erfid vor der oder den Leuchtdioden ein Streuelement angeordnet sein, das beispielsweise aus einer Spezialfolie besteht, die die Eigenscnaft hat das Licht immer mit einem ganz bestimmten Öffnungswinkel zu emittieren. Derartige Folien sind mit Öffnungswinkeln zwischen ±7,5° und ±45°erhältlich. So können Unterschiede in den Lichtkegeln der einzelnen Leuchtdioden ausgeglichen und die Aperturen geeignet geformt werden.

Vor allem aber wird die Betriebsdauer der Handlupe aufgrund des geringeren Stromverbrauchs von Leuchtdioden gegenüber Halogenlampen deutlich erhöht.

Zur Steuerung der Helligkeit der Beleuchtung ist es bevorzugt, Leuchtdioden abzuschalten; selbstverständlich kann aber auch die Versorgungsspannung der Leuchtdioden variiert oder getaktet werden.

Die Verlustleistung der Leuchtdioden kann ferner dazu benutzt werden, die Abstandsaufsätze auf eine hautfreundliche Temperatur zu erwärmen. Hierzu können die Abstandsaufsätze thermisch mit den Weißlicht-Leuchtdioden gekoppelt sein. Diese Ausbildung hat den weiteren Vorteil, daß keine Feuchtigkeit auf dem Deckglas kondensieren kann.

Weiterhin ist es bevorzugt, wenn zumindest die mit der untersuchten Person in Berührung kommenden Teile des erfindungsgemäßen Systems aus einem biokompatiblen Material bestehen. Derartige biokompatible Teile sind zum Beispiel Saphir für das Deckglas bzw. Deckfenster der Abstandsaufsätze. Die metallischen Teile können aus Chrom oder einer Chrom-Legierung bestehen oder chromiert sein.

Ferner ist es bevorzugt, wenn zumindest die Abstandsaufsätze sterilisierbar und insbesondere autoklavierbar sind. Will man eine Sterilisierung der Handlupe vermeiden und dennoch unter sterilen Bedingungen arbeiten, so ist es von Vorteil, wenn zumindest die Handlupe in einen sterilen Überzug einsetzbar ist, der insbesondere zwischen. Handstück und Abstandsaufsatz der Art eingelegt sein kann, daß er weder den Beleuchtungs- noch den Beobachtungsstrahlengang behindert, aber dennoch eine Berührung des sterilen Abstandsaufsatzes und des nicht sterilen Handstücks verhindert. Dabei ist es von besonderen Vorteil - wie bereits erwähnt -, wenn das Handstück und der Abstandsaufsatz magnetisch miteinander verbunden sind.

Weiterhin können in den Beleuchtungs - und/oder im Beobachtungsstrahlengang Filter und insbesondere Kantenfilter einbringbar und/oder eingebracht sein. Dabei können der oder die Filter Bestandteil der Streuscheibe(n), der Deckscheibe, der Optik oder ein eigenes Bauelement sein.

Das Griffteil kann dabei in bekannter Weise die Energieversorgung - Batterien oder aufladbare Akkus - für die Beleuchtungseinrichtung aufnehmen.

Weiterhin kann zwischen der Längsachse des Handstücks und der optische Achse der Lupenoptik ein Winkel von ca. 95°bis ca. 145°und insbesondere ca. 110°eingeschlossen sein. Hierdurch erhält man bei geringem Abstand des abgewinkelten Teils (Griffteils) im Bereich des auf die Haut aufgesetzten Kopfes einen vergleichsweise großen und damit ergonomisch günstigen Abstand in dem Bereich, in dem die Bedienungsperson das Griffteil hält.

### Kurze Beschreibung der Zeichnung

Die Erfindung wird nachstehend ohne Beschränkung des allgemeinen Erfindungsgedankens anhand von Ausführungsbeispielen unter Bezugnahme auf die Zeichnung exemplarisch beschrieben, auf die im übrigen hinsichtlich der Offenbarung aller im Text nicht näher erläuterten erfindungsgemäßen Einzelheiten ausdrücklich verwiesen wird. Es zeigen:

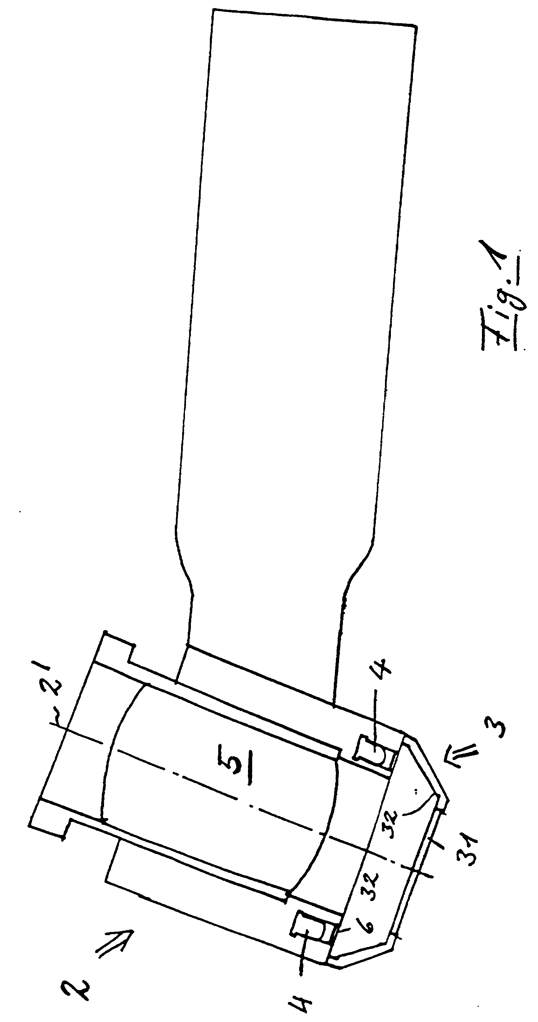

- Fig. 1: einen Querschnitt durch eine erfindungsgemäß ausgebildete Handlupe.

### Beschreibung eines Ausführungsbeispiels

Fig. 1 zeigt schematisch eine erfindungsgemäß ausgebildete Handlupe. Diese weist ein Griffteil 1 auf, in dem die Stromversorgung für die Beleuchtungseinrichtung angebracht ist. An dem Griffteil 1 ist ein Kopfstück 2 angebracht, dessen Achse 2' mit der Längsachse des Griffteils 1 einen Winkel von ca. 110° einschließt.

Auf das vordere Ende des Kopfteils 2 sind verschiedene Abstandsaufsätze 3 aufsetzbar, die z. B. magnetisch gehalten werden und eine unterschiedliche Erstreckung in Richtung der Achse 2' aufweisen. Die Abstandsaufsätze 3 an ihrem vordere Ende ein beispielsweise aus Saphir bestehendes Deckglas 31 auf.

In dem Kopfstück 2 ist ferner fest eine Beleuchtungseinrichtung angebracht, die bei dem gezeigten Ausführungsbeispiel Leuchtdioden 4 aufweist, die ringförmig um eine Lupenoptik 5 angeordnet sind. Bei den Leuchtdioden 4 handelt es sich um Weißlicht-Leuchtdioden. Das von den Leuchtdioden 4 ausgehende Lichbündel wird durch Streuelemente 6 hinsichtlich seiner Apertur geformt und von der mattierten Innenseite 32 des Abstandsaufsatzes 3 auf das Objektfeld gerichtet.

Die Lupenoptik 5 ist bei dem gezeigten Ausführungsbeispiel mittels eines Bajonetts auswechselbar. In der Zeichnung ist die Lupenoptik 5 als Einzellinse dargestellt, selbstverständlich können die Komponenten bei einer realen Ausführung aus mehreren Linsen mit sphärischen oder asphärischen Flächen bestehen.

## Patentansprüche

1. Dermatologie-Handlupe mit einem Handstück (1,2), mit einer Lichtquelle (4), die eine ringförmig angeordnete Beleuchtungseinheit für den zu untersuchenden Hautbereich aufweist, mit einer Lupenoptik (5) und mit einem ein Deckglas (31) an ihrem distalen Ende aufweisenden, wechselbaren Abstandsaufsatz (3), **dadurch gekennzeichnet, dass** die Beleuchtungseinheit als Lichtquelle eine Mehrzahl von ringförmig um die Lupenoptik (5) angeordnete Weißlicht-Leuchtdioden (4) aufweist und dass zur Erzielung einer Gleichmäßigkeit der Beleuchtung des zu untersuchenden Hautbereichs der Abstandsaufsatz (3) eine mattierte Fläche (32) an dessen Innenseite aufweist, wodurch das Licht der Weißlicht-Leuchtdioden (4) auf den zu beleuchtenden Hautbereich reflektiert wird.

2. Dermatologie-Handlupe nach Anspruch 1, **dadurch gekennzeichnet, dass** der Abstandsaufsatz (3) sterilisierbar und insbesondere autoklavierbar ist.

3. Dermatologie-Handlupe nach Anspruch 1 oder 2, **dadurch gekennzeichnet, dass** an dem Handstück (1,2) ein Kopfstück (2) angebracht ist, wobei dessen Achse (2') bzw. die optische Achse der Lupenoptik (5) einen Winkel von 95° bis 145° mit dem abgewinkelten Griffteil (1) bildet, und dass die Weißlicht-Leuchtdioden (4) in dem Kopfstück (2) angeordnet sind.

4. Dermatologie-Handlupe nach einem der Ansprüche 1 bis 3, **dadurch gekennzeichnet, dass** die Abstandsaufsätze (3) auf das Ende des Kopfteils (2) aufgesetzt sind, das im Betrielszustand dem zu untersuchenden Hantbereich zugewandt ist.

5. Dermatologie-Handlupe nach einem der Ansprüche 2 bis 4, **dadurch gekennzeichnet, dass** die Lupenoptik (5) in dem Kopfstück (2) angeordnet ist.

6. Dermatologie-Handlupe nach einem der Ansprüche 1 bis 5, **dadurch gekennzeichnet, dass** verschiedne Abstandsaufsätze (3) eine unerschiedliche Erstreckung in Richtung der optischen Achse (2') der Lupenoptik (5) aufweisen.

7. Dermatologie-Handlupe nach Anspruch 1 bis 6, **dadurch gekennzeichnet, dass** die Abstandsaufsätze (3) magnetisch am Handstück gehalten sind.

8. Dermatologie-Handlupe nach einem der Ansprüche 1 bis 7, **dadurch gekennzeichnet, dass** die Verbindungsstelle zwischen Handstück (1, 2) und Abstandsaufsatz (8), abgedichtet ist.

9. Dermatologie-Handlupe nach Anspruch 8, **dadurch gekennzeichnet, dass** die Abdichtung durch einen O-Ring erfolgt.

10. Dermatologie-Handlupe nach einem der Ansprüche 1 bis 9, **dadurch gekennzeichnet, daß** die Beleuchtungseinheit fest an dem Handstück (1,2) angebracht ist, und dass zur Einstellung der Größe des betrachteten Hautbereichs die Lupenoptik (5) wechselbar ist.

11. Dermatologie-Handlupe nach Anspruch 10, **dadurch gekennzeichnet, dass** Lupenoptiken (5) mit verschiedenen Vergrößerungen an dem Handstück (1, 2) anbringbar sind.

12. Dermatologie-Handlupe nach einem der Ansprüche 1 bis 11, **dadurch gekennzeichnet, dass** die Lupenoptik (5) wenigstens eine asphärische Fläche aufweist.

13. Dermatologie-Handlupe nach einem der Ansprüche 1 bis 12, **dadurch gekennzeichnet, dass** die Lupenoptik mittels eines Bajonettflansches an dem Handstück (1, 2) befestigt ist.

14. Dermatologie-Handlupe nach einem der Ansprüche 1 bis 13, **dadurch gekennzeichnet, dass** die Gleichmäßigkeit der Beleuchtung größer als 90 % ist.

15. Dermatologie-Handlupe nach einem der Ansprüche 1 bis 14, **dadurch gekennzeichnet, dass** zur definierten Änderung der Beleuchtungsstärke die Weißlicht-Leuchtdioden (4) einzeln oder in Gruppen schaltbar sind.

16. Dermatologie-Handlupe nach einem der Ansprüche 1 bis 15, **dadurch gekennzeichnet, dass** die Abstandsaufsätze (3) thermisch mit den Weißlicht-Leuchtdioden (4) gekoppelt sind, so dass sie durch die Verlustleistung der Weißlicht-Leuchtdioden (4) erwärmt werden.

17. Dermatologie-Handlupe nach einem der Ansprüche 1 bis 16, **dadurch gekennzeichnet, dass** zumindest die mit der untersuchten Person in Berührung kommenden Teile aus einem biokompatiblen Material bestehen.

18. Dermatologie-Handlupe nach Anspruch 17, **dadurch gekennzeichnet, dass** die metallischen Teile aus Chrom oder einer Chrom-Legierung bestehen oder chromiert sind.

19. Dermatologie-Handlupe nach einem der Ansprüche 1 bis 18, **dadurch gekennzeichnet, dass** zumindest das Handstück (1, 2) in einem sterilien Überzug einsetzbar ist.

20. Dermatologie-Handlupe nach Anspruch 19, **dadurch gekennzeichnet, dass** der sterile Überzug zwischen Handstück (1, 2) und Abstandsaufsatz (3) eingelegt ist.

21. Dermatologie-Handlupe nach einem der Ansprüche 1 bis 20,' **dadurch gekennzeichnet, dass** im Beleuchtungsund/oder im Beobachtungsstrahlengang Filter und insbesondere Kantenfilter einbringbar und/oder eingebracht sind.

22. Dermatologie-Handlupe nach Anspruch 21, **dadurch gekennzeichnet, dass** der oder die Filter Bestandteil der Streuscheibe(n), der Deckscheibe, der Optik oder ein eigenes Bauelement sind.

## Claims

1. Handheld dermatological magnifier with a handle unit (1,2), with a light source (4), which comprises a lighting unit arranged in a ring shape for illuminating the region of the skin to be examined, with a magnifying optical system (5) and with a replaceable spacing attachment (3), which comprises a covering glass (31) at its distal end, **characterised in that**, as the light source, the lighting unit provides a plurality of white-light light-emitting-diodes (4) arranged in a ring shape around the magnifying optical system (5) and that, in order to achieve uniformity of illumination of the region of the skin to be examined, the spacing attachment (3) provides a mat surface (32) on its interior, the light of the white-light light-emitting-diodes (4) being reflected onto the region of the skin to be illuminated.

2. Handheld dermatological magnifier according to claim 1, **characterised in that** the spacing attachment (3) is capable of being sterilised, in particular, in an autoclave.

3. Handheld dermatological magnifier according to claim 1 or 2, **characterised in that** a head component (2) is attached to the handle unit (1,2), wherein its axis (2') and/or the optical axis of the magnifying optical system (5) forms an angle from 95° to 145° relative to the angled handle component (1), and that the white-light light-emitting-diodes (4) are arranged in the head component (2).

4. Handheld dermatological magnifier according to any one of claims 1 to 3, **characterised in that** the spacing attachments (3) are fitted onto the end of the head component (2) facing in the operational condition towards the region of the skin to be examined.

5. Handheld dermatological magnifier according to any one of claims 2 to 4, **characterised in that** the magnifying optical system (5) is arranged in the head component (2).

6. Handheld dermatological magnifier according to any one of claims 1 to 5, **characterised in that** the different spacing attachments (3) provide a different extension in the direction of the optical axis (2') of the magnifying optical system (5).

7. Handheld dermatological magnifier according to claim 1 to 6, **characterised in that** the spacing attachments (3) are held onto the handle unit magnetically.

8. Handheld dermatological magnifier according to any one of claims 1 to 7, **characterised in that** the connecting point between the handle unit (1,2) and the spacing attachment (3) is sealed.

9. Handheld dermatological magnifier according to claim 8, **characterised in that** the seal is achieved by means of an O-ring.

10. Handheld dermatological magnifier according to any one of claims 1 to 9, **characterised in that** the lighting unit is firmly attached to the handle unit (1, 2), and that the magnifying optical system (5) can be replaced in order to adjust the magnification of the region of skin to be examined.

11. Handheld dermatological magnifier according to claim 10, **characterised in that** magnifying optical systems (5) with different magnifications can be attached to the handle unit (1,2).

12. Handheld dermatological magnifier according to any one of claims 1 to 11, **characterised in that** the magnifying optical system (5) provides at least one aspherical surface.

13. Handheld dermatological magnifier according to any one of claims 1 to 12, **characterised in that** the magnifying optical system is attached to the handle unit (1,2) by means of a bayonet flange.

14. Handheld dermatological magnifier according to any one of claims 1 to 13, **characterised in that** the uniformity of illumination is greater than 90%.

15. Handheld dermatological magnifier according to any one of claims 1 to 14, **characterised in that** the white-light light-emitting-diodes (4) can be connected individually or in groups in order to achieve a defined alteration of illuminance.

16. Handheld dermatological magnifier according to any one of claims 1 to 15, **characterised in that** the spacing attachments (3) are thermally coupled to the white-light light-emitting diodes (4) being heated by means of the heat loss from the white-light light-emitting-diodes (4).

17. Handheld dermatological magnifier according to any one of claims 1 to 16, **characterised in that** at least the components which come into contact with the person being examined are made from a biocompatible material.

18. Handheld dermatological magnifier according to claim 17, **characterised in that** the metallic components are made from chrome, a chrome alloy, or are chrome plated.

19. Handheld dermatological magnifier according to any one of claims 1 to 18, **characterised in that** the handle unit (1,2) at least can be inserted into a sterile cover.

20. Handheld dermatological magnifier according to claim 19, **characterised in that** the sterile cover is inserted between the handle unit (1,2) and the spacing attachment (3).

21. Handheld dermatological magnifier according to any one of claims 1 to 20, **characterised in that** filters, and in particular, edge filters can be introduced and/or are introduced into the optical lighting path or the optical observation path.

22. Handheld dermatological magnifier according to claim 21, **characterised in that** the filter(s) is/are a component of the dispersion glass(es), the cover glass(es), the optical system(s), or a separate component.

## Revendications

1. Loupe à main pour dermatologue comprenant un manche (1, 2), une source lumineuse (4) qui présente une unité d'éclairage disposée en anneau pour la zone de peau à examiner, une optique de loupe (5) et un chapeau de maintien à distance (3) interchangeable qui présente un verre couvre-objet (31) à son extrémité distale, **caractérisée en ce que** l'unité d'éclairage présente comme source lumineuse une pluralité de diodes électroluminescentes à lumière blanche (4) disposées en anneau autour de l'optique de loupe (5) et **en ce que**, pour assurer une uniformité de l'éclairage de la zone de peau à examiner, le chapeau de maintien à distance (3) présente une surface dépolie (32) sur sa face intérieure, de sorte que la lumière des diodes électroluminescentes à lumière blanche (4) est réfléchie vers la zone de peau à éclairer.

2. Loupe de dermatologue selon la revendication 1, **caractérisée en ce que** le chapeau de maintien à distance (3) est stérilisable et en particulier à l'autoclave.

3. Loupe de dermatologue selon la revendication 1 ou 2, **caractérisée en ce qu'**au manche (1, 2) est fixée une pièce de tête (2), l'axe (2') de cette pièce ou l'axe optique de l'optique de loupe (5) formant un angle de 95 ° à 145 ° avec la poignée coudée (1) et **en ce que** les diodes électroluminescentes à lumière blanche (4) sont disposées dans la pièce de tête (2).

4. Loupe de dermatologue selon une des revendications 1 à 3, **caractérisée en ce que**, dans l'état de fonctionnement, les chapeaux de maintien à distance (3) sont montés sur l'extrémité de la pièce de tête (2) qui est dirigée vers la zone de peau à examiner.

5. Loupe de dermatologue selon une des revendications 2 à 4, **caractérisée en ce que** l'optique de loupe (5) est disposée dans la pièce de tête (2).

6. Loupe de dermatologue selon une des revendications 2 à 5, **caractérisée en ce que** différents chapeaux de maintien à distance (3) présentent différentes dimensions selon la direction de l'axe optique (2') de l'optique de loupe (5).

7. Loupe de dermatologue selon la revendication 1 à 6, **caractérisée en ce que** les chapeaux de maintien à distance (3) sont fixés au manche par une action magnétique.

8. Loupe de dermatologue selon une des revendications 1 à 7, **caractérisée en ce que** la zone d'assemblage entre le manche (1, 2) et le chapeau de maintien à distance (3) est réalisée à joint étanche.

9. Loupe de dermatologue selon la revendication 8, **caractérisée en ce que** le joint étanche est réalisé au moyen d'une bague torique.

10. Loupe de dermatologue selon une des revendications 1 à 9, **caractérisée en ce que** l'unité d'éclairage est fixée rigidement au manche (1, 2) et **en ce que** l'optique de loupe (5) peut être interchangée pour le réglage de la dimension de la zone de peau observée.

11. Loupe de dermatologue selon la revendication 10, **caractérisée en ce que** des optiques de loupe (5) possédant différents grossissements peuvent être fixées au manche (1, 2).

12. Loupe de dermatologue selon une des revendications 1 à 11, **caractérisée en ce que** l'optique de loupe (5) présente au moins une surface asphérique.

13. Loupe de dermatologue selon une des revendications 1 à 12, **caractérisée en ce que** l'optique de loupe est fixée au manche (1, 2) au moyen d'un collet à baïonnette.

14. Loupe de dermatologue selon une des revendications 1 à 13, **caractérisée en ce que** l'uniformité de l'éclairage est supérieure à 90 %.

15. Loupe de dermatologue selon une des revendications 1 à 14, **caractérisée en ce que**, pour une modification définie de l'intensité de l'éclairage, les diodes électroluminescentes à lumière blanche (4) peuvent être mises en circuit individuellement ou par groupes.

16. Loupe de dermatologue selon une des revendications 1 à 15, **caractérisée en ce que** les chapeaux de maintien à distance (3) sont couplés thermiquement aux diodes électroluminescentes à lumière blanche (4), de sorte qu'ils sont chauffés par la puissance perdue des diodes électroluminescentes à lumière blanche (4).

17. Loupe de dermatologue selon une des revendications 1 à 16, **caractérisée en ce qu'**au moins les parties qui entrent en contact avec la personne à examiner sont composées d'une matière biocompatible.

18. Loupe de dermatologue selon la revendication 17, **caractérisée en ce que** les parties métalliques sont faites de chrome ou d'un alliage de chrome ou encore sont chromées.

19. Loupe de dermatologue selon une des revendications 1 à 18, **caractérisée en ce qu'**au moins le manche (1, 2) peut être emboîté dans une housse stérile.

20. Loupe de dermatologue selon la revendication 19, **caractérisée en ce que** la housse stérile est insérée entre le manche (1, 2) et le chapeau de maintien à distance (3).

21. Loupe de dermatologue selon une des revendications 1 à 20, **caractérisée en ce que** des filtres et, en particulier des filtres à arêtes peuvent être intercalés et/ou sont intercalés sur le trajet des rayons lumineux de l'éclairage et/ou sur le trajet des rayons lumineux de l'observation.

22. Loupe de dermatologue selon la revendication 21, **caractérisée en ce que** le ou les filtre(s) fait ou font partie du ou des diffuseur(s), du verre couvre-objet, de l'optique ou constitue(nt) un élément de construction distinct.
